# EUROPEAN PATENT SPECIFICATION

(11) **EP 2 515 860 B1**
(45) Date of publication and mention of the grant of the patent: **29.07.2015**
(21) Application number: 10805667.2
(22) Date of filing: 24.12.2010
(51) Int. Cl.: A61K 9/46, A61K 9/16, A61K 9/20

(54) **IMPROVED PHARMACEUTICAL COMPOSITIONS COMPRISING CEFDINIR**
VERBESSERTE PHARMAZEUTISCHE ZUSAMMENSETZUNGEN MIT CEFDINIR
COMPOSITIONS PHARMACEUTIQUES AMÉLIORÉES COMPRENANT CEFDINIR

(30) Priority: 25.12.2009 TR 200909785
(43) Date of publication of application: 31.10.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000261
(87) International publication number: WO 2011/078831

(56) References cited:
- WO-A1-2004/104010
- WO-A1-2006/106529
- US-A1- 2008 103 124
- DATABASE WPI Week 200630 Thomson Scientific, London, GB; AN 2006-285271 XP002620814, & CN 1 706 389 A (JINAN PINGZHI MEDICINE SCI TECH CO LTD) 14 December 2005 (2005-12-14)
- DATABASE WPI Week 200925 Thomson Scientific, London, GB; AN 2009-F19584 XP002620815, & CN 101 352 424 A (TIANJIN CENT MEDICINE CO LTD) 28 January 2009 (2009-01-28)

## Description

Present invention is related to water dispersible pharmaceutical dosage forms comprising cefdinir as active agent and methods for preparation of these.

### Background of the invention

Cefdinir molecule which is shown with Formula I was first disclosed in the patent numbered BE897864 and its chemical name is (6R,7R)-7-[[(2Z)-(2-amino-4-thiazolil)(hydroxyimino)acetyl] amino]-3-ethenyl-8-oxo-5-thia-1-azabicyclo[4.2.0]oct-2-en-2-carboxylic acid. The molecule which is a third generation cephalosporin is indicated for the treatment of several illnesses caused by gram positive and gram negative bacteria.

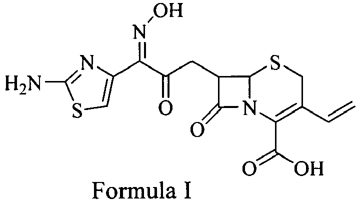

Cefdinir is an oral broad-spectrum and semisynthetic cephalosporin. Cefdinir physically appears as a white powder and is in white color. It is a molecule which has hydrophobic and non-polar character. For that reason, the solubility of cefdinir is very low in common organic solvents such as methanol, ethanol, acetonitrile and in water. Due to this property there are some problems while developing formulations comprising this molecule and in the bioavailability of the finished product.

The product sold in the market under the tradename OMNICEF^{®} is present in capsule and suspension forms. Clinic studies show that the bioavailability of the suspension product is 120% more than the bioavailability of the product in capsule form.

Although the suspension forms have higher bioavailability, use of this dosage form especially for pediatric and geriatric patients brings about the possibility of taking high and/or uncontrolled dose. Additionally, the fact that the suspensions have physical and chemical stability problems, they have short shelf life and high production costs and the fact that they cause problems while transporting and use are disadvantageous for the manufacturers.

Therefore, considering the disadvantages of suspension forms and the problems resulting from them, it is required to develop new dosage forms which have high bioavailabiliy but do not have any of said disadvantages.

Considering the prior art, it is necessary to develop; new pharmaceutical compositions comprising cefdinir with low water solubility that are stable and water dispersible, have long shelf life, easy to use and especially have high bioavailability.

The inventors have surprisingly found that the use of organic base in the dispersible powder, tablet, granule formulations is effective for solving the problems such as insolubility in water and low bioavailability which are present in the prior art.

### Detailed description of the invention

Present invention is related to the use of organic base in the water dispersible powder, tablet, and granule formulations comprising cefdinir as active agent. Surprisingly, as a result of the studies for development of water dispersible powder, tablet, granule forms, it is seen that the use of organic base in the formulation has an effect on solving the insolubility problem of cefdinir in water.

Therefore, it was observed that adding organic base into the formulation influenced the solubility of cefdinir, which is hydrophobic in water, to a considerable extent. Therefore, it was seen that water dispersible powder, tablet, granule formulations having both the advantages of tablet and suspension forms was developed and it was seen that these forms have a higher bioavailability compared to tablet formulations.

The term "water dispersible powder, tablet and granule" comprises effervescent tablets, effervescent granules, effervescent powders, water dispersible tablets, water dispersible powders and water dispersible granules, water soluble tablets, water soluble powders, water soluble granules.

Organic base that is used in the formulation is 1-deoxy-1-methylamino-sorbitol or tris(hydroxymethyl)aminomethane.

Cefdinir which can be used in the water dispersible powder, tablet and granule formulations comprising organic base of the present invention can be present in the form of its solvates, hydrates, enetiomers, racemates, organic salts, inorganic salts, polymorphs, crystal and amorphous forms or in free form and/or as a combination of these.

In said formulation, in addition to cefdinir and organic base several other excipients such as binders, lubricants, humectants, disintegrants, basic agents, acidic agents, sweeteners and optionally effervescent couple can be used.

Binder which can be used in the water dispersible powder, tablet and granule formulations comprising organic base according to the present invention can be selected from, but not limited with, a group comprising ethyl cellulose, gelatine, hydroxy ethyl cellulose, hydroxy methyl cellulose, hydroxypropyl cellulose, hypromellose, magnesium aluminium silicate, methyl cellulose, povidone.

Lubricant which can be used in the water dispersible powder, tablet and granule formulations comprising organic base according to the present invention can be selected from, but not limited with, a group comprising calcium stearate, magnesium stearate, polyethylene glycol, PEG6000, polyvinyl alcohol, potassium benzoate, sodium benzoate.

Humectant which can be used in the water dispersible powder, tablet and granule formulations comprising organic base according to the present invention can be selected from, but not limited with, a group comprising anhydrous sodium sulphate, silica gel and potassium carbonate. Disintegrant which can be used in the water dispersible powder, tablet and granule formulations comprising organic base according to the present invention can be selected from, but not limited with a group comprising carboxymethyl cellulose calcium, carboxymethyl cellulose sodium, microcrystalline cellulose, silicon dioxide, croscarmellose sodium, crospovidone, hydroxypropyl cellulose, methyl cellulose, povidone, magnesium aluminium silicate, starch or a combination thereof.

Diluent which can be used in the water dispersible powder, tablet and granule formulations comprising organic base according to the present invention can be selected from, but not limited with, a group comprising calcium carbonate, calcium sulfate, dibasic calcium phophate, tribasic calcium sulfate, calcium sulfate, microcrystalline cellulose, lactose, magnesium carbonate, magnesium oxide, maltodextrine, maltose, mannitol, sodium chloride, sorbitol, starch, xylitol or a combination thereof.

Basic agent which can be used in the water dispersible powder, tablet and granule formulations comprising organic base according to the present invention can be selected from, but not limited with, a group comprising potassium carbonate, potassium citrate, potassium hydroxide, sodium carbonate, sodium bicarbonate or combinations thereof.

Acidic agent which can be used in the water dispersible powder, tablet and granule formulations comprising organic base according to the present invention can be selected from, but not limited with, a group comprising acetic acid, citric acid, lactic acid, malic acid, phosphoric acid, propionic acid, tartaric acid or combinations thereof.

Sweetener which can be used in the water dispersible powder, tablet and granule formulations comprising organic base according to the present invention can be selected from, but not limited with, a group comprising acesulfame, aspartamate, dextrose, fructose, glucose, lactitol, maltitol, maltose, sorbitol, saccharide, sodium saccharide, sodium cyclamate, sucralose, sodium chloride, potassium chloride, sucrose, xylitol or combinations thereof.

Effervescent couple which can be used in the water dispersible powder, tablet and granule formulations comprising organic base of the present invention can be selected from, but not limited with, organic acids such as citric acid, tartaric acid, malic acid, furmaric acid etc and organic bases such as sodium carbonate, sodium hydrogen carbonate, potassium carbonate, potassium hydrogen carbonate etc.

In water dispersible powder, tablet and granule formulations comprising organic base according to the present invention, 1-3500 mg of cefdinir or its pharmaceutically acceptable salts, hydrates, solvates and/or a combination of these in an amount equivalent to that can be used.

In water dispersible powder, tablet and granule formulations comprising organic base according to the present invention, with respect to the total weight of the unit dose; 5-55% of cefdinir or its pharmaceutically acceptable solvates, hydrates, enantiomers, racemates, organic salts, inorganic salts, polymorphs, crystal/amorphous forms or a combination of these, 1-30% organic base, 1-35% binder, 0.1-3 % lubricant, % 0.1-5% sweetener, 0.1-6 % coloring and/or flavouring agent and optionally 0-85% effervescent couple can be present.

In another aspect present invention relates to processes used for the preparation of water dispersible powder, tablet and granule formulations comprising cefdinir as active agent, organic base and pharmaceutically acceptable excipients.

Accordingly, process that is used in the present invention comprises granulation of cefdinir with conventional dry and/or wet granulation methods known in the art or mixing cefdinir and other excipients with a dry blending method and optionally pressing them in tablet form or filling them into sachets.

Water dispersible tablets comprising organic base in accordance with the present invention can be prepared according to the following examples provided that they are not limited by these examples.

### EXAMPLE 1: Formulation and process for preperation of effervescent granules

| | % amount in unit dose |
|---|---|
| Cefdinir | 35% |
| Organic base | 10% |
| Citric acid | 30% |
| Sodium hydrogen carbonate | 18% |
| Binder | 3% |
| Sweetener | 1.75% |
| Lubricant | 0.75% |
| Coloring agent | 1.25% |
| Flavoring agent | 1% |

Formulation is obtained by granulation of sodium hydrogen carbonate and cefdinir with aqueous solution of organic base and then mixing the formed granules with citric acid and sweetener. The formed mixture is then granulated with a solution of binder. The granule obtained after this step is mixed with lubricant, coloring agent, sweetener and flavoring agent and optionally the final mixture is compressed in the form of tablets.

### EXAMPLE 2: Formulation and process for preperation of water dispersible granules

| | % amount in unit dose |
|---|---|
| Cefdinir | % 40 |
| Organic base | % 20 |
| Binder | % 25 |
| Sweetener | % 5.0 |
| Lubricant | % 1.5 |
| Coloring agent | % 3.5 |
| Flavoring agent | % 5.0 |

Formulation can be obtained by granulation of cefdinir with aqueous solution of organic base and then mixing the formed granules with sweetener. The formed mixture is then granulated with a solution of binder. The granule obtained after this step is mixed with lubricant, coloring agent, sweetener and flavoring agent and optionally the final mixture is filled into sachets.

In another aspect present invention relates to use of water dispersible powder, tablet and granule formulations comprising cefdinir and in addition to that pharmaceutically acceptable excipients for the treatment of infections caused by gram positive and gram negative bacteria.

## Claims

1. A pharmaceutical composition comprising cefdinir and organic base, wherein said composition is in effervescent form; and wherein the organic base is selected from a group comprising 1-deoxy-1-methylamino-sorbitol and tris(hydroxymethyl)aminomethane.

2. The pharmaceutical composition according to claim 1, wherein said composition can be present in powder, tablet and/or granule form.

3. The pharmaceutical composition according to claim 1-2, wherein said composition can be in the form of effervescent powder, effervescent tablet, effervescent granule, water dispersible powder, water dispersible granule, water dispersible tablet, water soluble tablet, water soluble granule, water soluble powder.

4. The pharmaceutical composition according to claim 1 wherein cefdinir can be in the form of its solvates, hydrates, enantiomers, racemates, organic salts, inorganic salts, polymorphs, crystal and amorphous forms or in free form and/or a combination thereof.

5. The composition according to claim 1 wherein said composition comprises pharmaceutically acceptable excipients in addition to cefdinir that is used as an active agent; preferably wherein the pharmaceutically acceptable excipients are wherein binders, lubricants, humectants, diluents, disintegrants, basic agents, acidic agents, sweeteners and optionally effervescent agents.

6. The pharmaceutical composition according to claim 5, wherein binder is selected from a group comprising ethyl cellulose, gelatine, hydroxyethyl cellulose, hydroxymethyl cellulose, hydroxypropyl cellulose, hypromellose, magnesium aluminium silicate, methyl cellulose, povidone.

7. The pharmaceutical composition according to claim 5 wherein lubricant is selected from a group comprising calcium stearate, magnesium stearate, polyethylene glycol, PEG 6000, polyvinyl alcohol, potassium benzoate, sodium benzoate.

8. The pharmaceutical composition according to claim 5 wherein humectant is selected from a group comprising sodium sulfate, silica gel, potassium carbonate.

9. The pharmaceutical composition according to claim 5 wherein disintegrant is selected from a group comprising carboxymethyl cellulose calcium, carboxymethyl cellulose sodium, microcrystalline cellulose, silicon dioxide, croscarmellose sodium, crospovidon, hydroxypropylcellulose, methyl cellulose, povidone, magnesium aluminium silicate, starch or a combination thereof.

10. The pharmaceutical composition according to claim 5 wherein diluent is selected from a group comprising calcium carbonate, calcium sulfate, dibasic calcium phosphate, tribasic calcium phosphate, calcium sulfate, microcrystalline cellulose, lactose, magnesium carbonate, magnesium oxide, maltodextrin, maltoz, mannitol, sodium chloride, sorbitol, starch, xylitol or combinations thereof.

11. The pharmaceutical composition according to claim 5 wherein basic agent is selected from a group comprising potassium carbonate, potassium bicarbonate, potassium citrate, potassium hydroxide, sodium carbonate, sodium bicarbonate, or combinations thereof; wherein acidic agent is selected from a group comprising acetic acid, citric acid, lactic acid, malic acid, phosphoric acid, propionic acid, tartaric acid or combinations thereof.

12. The pharmaceutical composition according to claim 5 wherein sweetener is selected from a group comprising acesulfame, aspartamate, dextrose, fructose, glucose, lactitol, maltitol, maltose, sorbitol, saccharine, saccharine sodium, sodium cyclamate, sucralose, xylitol, sodium chloride, potassium chloride or combinations thereof

13. The pharmaceutical composition according to claim 1-12 wherein with respect to the total weight of the unit dose; 5-55% of cefdinir or pharmaceutically acceptable solvates, hydrates, enantiomers, racemates, organic salts, inorganic salts, polymorphs, crystal/amorphous forms, 1-30% organic base, 1-35% binder, 0.1-3 % lubricant, % 0.1-6% sweetener, 0.1-7 % coloring and/or flavouring agent and optionally 0-85% effervescent couple are present.

14. A process for the preparation of the formulations according to claims 1-13 wherein said process comprises granulation of cefdinir with conventional dry and/or wet granulation methods known in the art or by mixing cefdinir and other excipients with a dry blending method and optionally compressing them in tablet form or filling them into sachets.

15. The composition according to any claims 1-13 for use in the treatment of infections caused by gram positive and gram negative bacteria.

## Patentansprüche

1. Eine pharmazeutische Zusammensetzung, die Cefdinir und organische Base beinhaltet, wobei die genanntezusammensetzung in Brauseform ist und wobei die organische Base aus einer Gruppe bestehend aus 1-Deoxy-1-methylamino-Sorbit und Tris (hydroxymethyl) aminomethan ausgewählt wird.

2. Die pharmazeutische Zusammensetzung nach Anspruch 1, wobei die genanntezusammensetzung in Pulver, Tabletten und / oder Granulatform vorgelegt werden kann.

3. Die pharmazeutische Zusammensetzung nach Anspruch 1-2, wobei die genanntezusammensetzung in Form von Brausepulver, Brausetablette, Brausegranulat, dem wasserdispergierbaren Pulver, dem wasserdispergierbaren Granulat, der wasserdispergierbaren Tablette, der wasserlöslichen Tablette, dem wasserlöslichen Granulat, dem wasserlöslichen Pulver vorliegen kann.

4. Die pharmazeutische Zusammensetzung nach Anspruch 1, wobei Cefdinir in Form seiner Solvats, Hydraten, Enantiomeren, Racematen, organischen Salzen, anorganischen Salzen, Polymorphen, kristall- und amorphen Formen oder in freier Form und / oder eine Kombination davon vorliegen kann.

5. Die Zusammensetzung nach Anspruch 1, wobei die genanntezusammensetzung pharmazeutisch annehmbare Hilfsstoffe neben Cefdinir beinhaltet, das als aktives Mittel verwendet wird, wobei die pharmazeutisch annehmbare Hilfsstoffe vorzugsweise Bindemittel, Gleitmittel, Feuchthaltemittel,Verdünnungsmittel, Sprengmittel, Basismittel, saure Mittel, Süßungsmittel und gegebenenfalls Brausemittel sind.

6. Die pharmazeutische Zusammensetzung nach Anspruch 5, wobei das Bindemittel aus einer Gruppe bestehend aus Ethylcellulose, Gelatine, Hydroxyethylcellulose, Hydroxymethylcellulose, Hydroxypropylcellulose, Hypromellose, Magnesiumaluminiumsilikat, Methylcellulose und Povidon ausgewählt wird.

7. Die pharmazeutische Zusammensetzung nach Anspruch 5, wobei Schmierstoff aus einer Gruppe bestehend aus Calciumstearat, Magnesiumstearat, Polyethylenglykol, PEG 6000, Polyvinylalkohol, Kaliumbenzoat, Natriumbenzoat ausgewählt wird.

8. Die pharmazeutische Zusammensetzung nach Anspruch 5, wobei Feuchthaltemittel aus einer Gruppe bestehend aus Natriumsulfat, Kieselgel, Kaliumcarbonat ausgewählt wird.

9. Die pharmazeutische Zusammensetzung nach Anspruch 5, wobei Sprengmittel aus einer Gruppe bestehend aus Carboxymethylcellulosecalcium, Carboxymethylcellulose-Natrium, mikrokristalliner Cellulose, Siliciumdioxid, Croscarmellose-Natrium, Crospovidon, Hydroxypropylcellulose, Methylcellulose, Povidon, Magnesiumaluminiumsilikat, Stärke oder eine Kombination davon ausgewählt wird.

10. Die pharmazeutische Zusammensetzung nach Anspruch 5, wobei Verdünnungsmittel aus einer Gruppe bestehend aus Calciumcarbonat, Calciumsulfat, zweibasischenCalciumphosphat, dreibasischen Calciumphosphat, Calciumsulfat, der mikrokristallinen Cellulose, Lactose, Magnesiumcarbonat, Magnesiumoxid, Maltodextrin, Maltoz, Mannit, Natrium Chlorid, Sorbit, Stärke, Xylitol oder Kombinationen davon ausgewählt wird.

11. Die pharmazeutische Zusammensetzung nach Anspruch 5, wobei basisches Mittel aus einer Gruppe bestehend aus Kaliumcarbonat, Kaliumbicarbonat, Kaliumcitrat, Kaliumhydroxid, Natriumcarbonat, Natriumbicarbonat, oder Kombinationen davon ausgewählt wird; wobei saures Mittel aus einer Gruppe bestehend aus Essigsäure, Zitronensäure, Milchsäure , Äpfelsäure , Phosphorsäure, Propionsäure, Weinsäure oder Kombinationen davon ausgewählt wird.

12. Die pharmazeutische Zusammensetzung nach Anspruch 5, wobei Süßungsmittel aus einer Gruppe bestehend aus Acesulfam, Aspartamate, Dextrose, Fructose, Glucose, Lactitol, Maltitol, Maltose, Sorbit, Saccharin, Saccharin-Natrium, Natrium-Cyclamat, Sucralose, Xylit,Natriumchlorid,Kaliumchlorid oder Kombinationen davon ausgewählt wird.

13. Die pharmazeutische Zusammensetzung nach Anspruch 1-12, wobei in Bezug auf das Gesamtgewicht der Einheitsdosis; 5-55% von Cefdinir oder pharmazeutisch annehmbaren Solvaten, Hydraten, Enantiomeren, Racematen, organischen Salzen, anorganischen Salzen, Polymorphen, Kristall/amorphen Formen, 1-30% der organischen Base, 1-35% des Bindemittels, 0,1-3% des Gleitmittels,% 0,1 - 6% des Süßstoffes, 0,1-7% des Farbstoffes und / oder Aromastoffes und wahlweise 0-85% des Brausepaares vorhanden sind.

14. Ein Verfahren zur Herstellung der Formulierungen nach den Ansprüchen 1-13,wobei das genannte Verfahren das Granulieren des Cefdinir durch die im Stand der Technik bekannten üblichen trockenen und/oder nassen Granulierungsverfahren oder durch Mischen von Cefdinir und anderen Hilfsstoffen mit dem Trockenmischverfahren und gegebenenfalls Zusammenpressen in Form von Tabletten oder Einfüllen in Beuteln beinhaltet.

15. Die Zusammensetzung nach einem der Ansprüche 1-13 zur Verwendung bei der Behandlung von Infektionen, die durch Gram-positive und Gram-negative Bakterien verursacht werden.

## Revendications

1. Une composition pharmaceutique comprenant cefdinir et la base organique dans laquelle ladite composition est sous la forme effervescente ; et dans laquelle la base organique est choisie d'un groupe comprenant 1-désoxy-1-méthylamino le sorbitol et le tris(hydroxyméthyl)aminométhane.

2. La composition pharmaceutique selon la revendication 1, dans laquelle ladite composition peut se présenter sous la forme de poudre, d'un comprimé et/ou sous la forme de granulés.

3. La composition pharmaceutique selon les revendications 1-2, dans lesquelles ladite composition peut se présenter sous la forme d'une poudre effervescente, d'un comprimé effervescent, d'un granule effervescent, d'une poudre dispersible dans l'eau, d'un granule dispersible dans l'eau, d'un comprimé dispersible dans l'eau, d'un comprimé soluble dans l'eau, d'un granule soluble dans l'eau, d'une poudre soluble dans l'eau.

4. Une composition pharmaceutique selon la revendication 1, dans laquelle, cefdinir peut être sous la forme de son solvates, hydrates, énantiomères, racémates, sels organiques, sels inorganiques, polymorphes, sous la forme cristalline et amorphe ou bien sous la forme libre et/ou d'une combinaison de ceux-ci.

5. La composition selon la revendication 1, dans laquelle ladite composition comprend les excipients pharmaceutiquement acceptables en plus de cefdinir qui est utilisé en tant qu'agent actif; de préférence dans laquelle les excipients pharmaceutiquement acceptables sont des liants, des lubrifiants, des humectants, des diluants, des désintégrants, des agents basiques, des agents acides, des édulcorants et éventuellement des agents effervescents.

6. La composition pharmaceutique selon la revendication 5, dans laquelle le liant est choisi d'un groupe comprenant l'éthylcellulose, la gélatine, l'hydroxyéthylcellulose, l'hydroxyméthylcellulose, l'hydroxypropylcellulose, l'hypromellose, le silicate de magnésium et d'aluminium, la méthylcellulose, la povidone.

7. La composition pharmaceutique selon la revendication 5, dans laquelle le lubrifiant est choisi d'un groupe comprenant le stéarate de calcium, le stéarate de magnésium, le polyéthylèneglycol, PEG 6000, l'alcool de polyvinyle, le benzoate de potassium, le benzoate de sodium.

8. La composition pharmaceutique selon la revendication 5, dans laquelle l'humectant est choisi d'un groupe comprenant du sulfate de sodium, le gel de silice, le carbonate de potassium.

9. La composition pharmaceutique selon la revendication 5, dans laquelle le désintégrant est choisi d'un groupe comprenant la carboxyméthylcellulose calcique, la carboxyméthylcellulose sodique, la cellulose microcristalline, le dioxyde de silicium, le croscarmellose sodique, la crospovidon, l'hydroxypropylcellulose, la méthylcellulose, la povidone, le silicate de magnésium et d'aluminium, de l'amidon ou une combinaison de ceux-ci.

10. La composition pharmaceutique selon la revendication 5, dans laquelle le diluant est choisi d'un groupe comprenant le carbonate de calcium, le sulfate de calcium, le phosphate de calcium dibasique, le phosphate de calcium tribasique, le sulfate de calcium, la cellulose microcristalline, le lactose, le carbonate de magnésium, l'oxyde de magnésium, la maltodextrine, le maltose, le mannitol, le chlorure de sodium, le sorbitol, l'amidon, le xylitol ou les combinaisons de ceux-ci.

11. La composition pharmaceutique selon la revendication 5, dans laquelle l'agent basique est choisi d'un groupe comprenant le carbonate de potassium, le bicarbonate de potassium, le citrate de potassium, l'hydroxyde de potassium, le carbonate de sodium, le bicarbonate de sodium, ou des combinaisons de ceux-ci ; dans laquelle l'agent acide est choisi d'un groupe comprenant l'acide acétique, l'acide citrique, l'acide lactique, l'acide malique, l'acide phosphorique, l'acide propionique, l'acide tartrique ou les combinaisons de ceux-ci.

12. La composition pharmaceutique selon la revendication 5, dans laquelle l'édulcorant est choisi d'un groupe comprenant l'acésulfame, l'aspartame, le dextrose, le fructose, le glucose, le lactilol, le maltilol, le maltose, le sorbitol, la saccharine de sodium, le cyclamate de sodium, le sucralose, le xylitol, le chlorure de sodium, le chlorure de potassium ou les combinaisons de ceux-ci.

13. La composition pharmaceutique selon les revendications 1-12, dans lesquelles par rapport au poids total de la dose unitaire; cefdinir ou des solvates pharmaceutiquement acceptables, leurs hydrates, des énantiomères, des racémates, des sels organiques, des sels inorganiques, des polymorphes, des formes cristaux/amorphes est présent à 5-55%, la base organique à 1-30%, le liant à 1-35%, le lubrifiant à 0.1-3 %, l'édulcorant à 0.1-6%, le colorant et/ou l'agent aromatisant à 0.1-7% et éventuellement le couple effervescent à 0-85%.

14. Un procédé pour la préparation des formulations selon les revendications 1-13, dans lesquelles ledit procédé comprend la granulation de cefdinir avec des méthodes de granulation par voie sèche et/ou humide conventionnelles connues dans l'art ou par mélanger le cefdinir et d'autres excipients avec un procédé de mélange à sec et éventuellement les pressant sous les forme de comprimés et les remplissant dans les sachets.

15. La composition selon quelconque des revendications 1-13 pour l'utilisation dans le traitement des infections causés par les bactéries à Gram positives et à Gram négatives.
